# EUROPEAN PATENT SPECIFICATION

(11) **EP 1 615 626 B1**
(45) Date of publication and mention of the grant of the patent: **14.10.2009**
(21) Application number: 04729172.9
(22) Date of filing: 23.04.2004
(51) Int. Cl.: A61K 9/28, A61K 9/20

(54) **TABLET WITH COLOURED CORE**
TABLETTE MIT GEFÄRBTEM KERN
COMPRIME A NOYAU COLORE

(30) Priority: 24.04.2003 GB 0309343
(43) Date of publication of application: 18.01.2006
(73) Proprietor: JAGOTEC AG, 4132 Muttenz (CH)
(72) Inventor: VERGNAULT, Guy, F-68680 Kembs, Loechle (FR); GRENIER, Pascal, 68510 Kappelen (FR); DRAGAN, Christophe, F-68510 Geispitzen (FR)
(74) Representative: Bassil, Nicholas Charles
(86) International application number: PCT/IB2004/001702
(87) International publication number: WO 2004/093850

(56) References cited:
- EP-A- 0 519 099
- GB-A- 860 708
- GB-A- 874 586
- US-A- 3 048 526
- US-A- 3 125 491

## Description

The present invention is related to a medicament in the form of a tablet having a core containing an active agent and a compression coating surrounding the core.

Medicaments in the form of tablets and containing a core comprising active agent and a coating surrounding the core are well known in the art. Coatings are employed around active-containing cores for a variety of reasons. For example, they may effectively protect the active substance from aggressive physiological media prior to the delivery of the medicament to the preferred site of absorption, or they may be employed as a means of modulating the release of the active agent from the core. Coatings may also be employed for aesthetic purposes such as to include a flavourant as a means of masking a bitter-tasting active agent or excipients, or they may be used to impart colour or combinations of colour in order to act as a visual cue to a patient as to the nature of the medicament that is being taken, or as a means of branding.

Compression coating is one means of providing a medicament with a functional and/or aesthetic coating. Such coatings may be applied using all manner of industrial press-coating equipment well known in the art. Medicaments may be formed by adding a part of the coating material as a granulate into a die, and tamping the powder to form a base. Thereafter, an active agent-containing core is added to the die and sits on the tamped powder. Finally, the remaining coating material is introduced into the die and this material is then compressed to form a hard coating surrounding the core. Such a process may be carried out automatically using commercially available press-coating equipment available from manufactures such as Killian or Manesty, or the like.

Using properly calibrated equipment, it is possible to ensure that essentially all compression coated unit dosage forms formed by compression coating techniques are formed with appropriately positioned, and intact cores. However, it is possible that some cores in a batch will not be correctly positioned in dies or that during compression a core may be broken. Still further, it is also possible, particularly in processes wherein cores are formed prior to their being coated, that core material may contaminate a die such that some of the core material finds its way into admixture with the coating material.

In any of these ways, active substance may find itself displaced from its intended position located in the core. If this occurs, the release of some of the active substance may not be correctly modulated or controlled by the coating such that the release kinetics of the active are incorrect for efficacious treatment. Also, when an active substance is an unpleasant-tasting material, any active substance finding its way on or near the surface of the medicament, or which is not sufficiently coated because of a displacement of the core, will be sensorially offensive to patients.

Careful calibration of press-coater machinery, and appropriate in-process control of batches of dosage forms, will generally eliminate any imprecision in the positioning of a core in a die, and ensure that many broken cores, or core-material contaminating the coating will be avoided or at least detected. However, such in-process control generally involves an operator having to cut open a random sample of the dosage forms and measure core positions, or subject the cut dosage forms to visual inspection to inspect the integrity of the core. Such in-process control is laborious, and may not pick-up the presence of small contaminant amounts of core material that may make its way into the coating. Accordingly, there remains a need to develop improved aids to in-process control.

The applicant has now found that by taking the expedient of adding a colourant or other excipients to the core, one can use non-invasive means, such as visible or higher energy radiation means, to locate the position and examine the integrity of a core in a coating. Furthermore, provided a colourant or excipient is used that provides a detectable contrast to the coating material, inspection with the naked eye a radiation source will detect any core material that is contaminating the coating at or near its surface.

The utilization of one or more colourants in medicaments is known in the art. Indeed, there are commercially available capsules, particularly for use in over-the-counter medicaments, which consist of transparent, or substantially transparent capsules containing a plurality of coloured or differently coloured beads containing medicament. However, such colouration is used to enable a user to readily identify a particular type or brand of medicament. There is also a perception of medicinal efficacy, and of course, such dosage forms are aesthetically pleasing to the eye. Also, such medicaments employ transparent coatings, such as soft-gelatin coatings.

US 3,125,491 describes a chewable hematinic vitamin tablet composed of a two-layered tablet, one of those layers consisting of a sugar compression coated core containing a form of vitamin C, the other layer consisting of a granulation of therapeutic ferrous iron. Pharmaceutically acceptable dyes may be used in the table to produce two-colour and two-tone tablets.

US 3,048,526 describes a tablet containing segregated quantities of the same or different ingredients, in particular a compressed coated tablet with a different colour in the core than in the marble. However, the marble does not completely cover the core.

EP 519,099 relates to a direct compression cyclophosphamide tablet in which a colourant may be present in the external coat.

GB 874,586 describes compression coated tablets that can contain dyes in the tablet core and the coating. However, this document does not address the present problem and therefore does not recognize the significance of having a detectable contrast between the core and the coating.

Accordingly, the invention provides in a first aspect a tablet comprising a core containing active substance and a compression coating surrounding the core, wherein the core contains a colourant selected from the group consisting of titanium dioxide, calcium sulphate, magnesium oxide, aluminium silicate, aluminium hydroxide and iron oxide or an excipient that is opaque to x-rays such that when the tablet is exposed to penetrating radiation the core is contrasted with the coating and is visible through the coating.

In another aspect, the invention provides the use of a colouring agent selected from the group consisting of titanium dioxide, calcium carbonate, calcium sulphate, magnesium oxide, aluminium silicate, aluminium hydroxide, talc and iron oxide or an excipient opaque to x-ray radiation in the core of a press-coated tablet comprising a core containing an active substance, and a coating surrounding said core, as a means for determining that the core is correctly located within the coating.

Tablets of the present invention may be easily examined during in-process control using appropriate radiation detection means. The radiation source may be a high energy radiation source such as x-ray radiation or it may be a visible light source. For example, as tablets are ejected from a press-coater, they can pass through a visible light source, and the contrasting core can be detected with a suitable detector, such as a camera. Alternatively, high energy radiation such as x-ray radiation can be focused on the tablets and a core containing excipients contained in the core that are opaque to x-ray radiation (such as barium sulphate) will contrast the core. The detector will display an image of the core that can be relayed to an operator, or which can be processed automatically with a computer, such that if a damaged or incorrectly placed core is detected the tablet manufacturing process can be stopped and the problem addressed. The invention therefore enables cheaper, more efficient in-process control, and permits of more controlled administration of drug substances to patients.

The present invention is particularly usefully employed in medicaments that are foul tasting. The present invention is also particularly useful to administer active substances in a time-controlled manner. In such dosage forms if the release rate of the active agent is dependent on the core thickness, the precise location of the active-containing core within the coating will be an important consideration. A damaged core, or an imprecisely placed core, may cause the active substance to be released in an uncontrolled manner, and a patient may receive an incorrect dose of active substance.

However, if by means of the present invention a core can be reliably and accurately positioned within a coating, tablets having precisely defined release profiles can be obtained. This is particularly advantageous when a tablet is adapted to release a drug substance only after a defined lag time after administration. Such dosage forms are useful to target particularly narrow absorption windows in the GI tract, or in targeting small locally affected areas of the GI tract or bowel. Such dosage forms are also useful for releasing an active substance with a lag time to coincide with a nocturnal circadian rhythm that is responsible for causing symptoms in patients. An example of this is rheumatoid arthritis. This condition is responsible for causing joint pain in patients in the early morning after waking. A cause of these symptoms however, is believed to be the secretion of IL-6 in the early hours, e.g. 2am to 4am. Being able to take a tablet at a convenient hour before bedtime, and having the drug released after a lag time to coincide with IL-6 secretion would have enormous advantages compared with a dosage form that has to be taken only upon waking when the symptoms are already apparent.

Colourants for use in the present invention are the pigments titanium dioxide, calcium sulphate, magnesium oxide, magnesium carbonate, aluminium silicate, aluminium hydroxide, and iron oxide. The amount of colorant used depends upon the appearance desired and can be adjusted accordingly.

A preferred colourant for use in the present invention is red ferric oxide.

Colourants may be present in a range of from 0.1% to about 1.75%, preferably of from 0.2% to about 1.5%, and in certain preferred embodiments from 0.25% to 1.0% based on the total weight of the tablet.

Other excipients may be employed in the core that render the core opaque to high energy radiation, such as x-ray radiation. Any substance useful for this purpose and which is pharmaceutically acceptable can be employed. Barium sulphate is an example of such an excipient. Such excipients may be employed in amounts ranging from 0.1 to 2% by weight based on the weight of the core.

The skilled person will appreciate that any active agent or combination of active agents may be employed in tablets of the present invention.

As stated above, a wide variety of drug substances may be employed in the present invention. Drugs for treating conditions the symptoms of which result from nocturnal circadian rhythms are particularly suitable. Accordingly, drugs for treating incontinence, sleep disorders, apnoea, asthma, epilepsy, bronchitis, parkinsonism, rheumatoid arthritis, allergic rhinitis and ischaemic heart diseases, cluster and migraine headache, congestive heart failure, and depression are particularly suitable for use in tablets according to the present invention. Further, drug substances that are metabolized by cytochrome P450 are also particularly suitable, they include:-

Amitriptyline, caffeine, clomipramine, clozapine, fluvoxamine, haloperidol, imipramine, mexilitine, oestradiol, olanzepine, paracetamol, propranolol, tacrine, theophylline, warfarin, Bupropion, Cyclophosphamide, Celecoxib, Diclofenac, Flubiprofen, Ibuprofen, glimepirideindome, thacin, naproxen, phenytoin, piroxicam, tenoxicam, citalopram, diazepam, lansoprazole, omeprazole, pantoprozole, propanolol, topiramate, Alpranolol, chlorpromazine, clomipramine, codeine, Desipramine, dextromethorphan, diphenhydramine, donepezil, flecainide, fluoxetine, labetalol, Methadone, metoprolol, mianserin, nortripyline, ondansetron, oxprenolol, oxycodone, paroxetine, perhehexilene, pethidine, promethazine, risperdone, thioridazine, ticlopidine, timolol, trimipramine, venlafaxine, paracetamol, alprazolam, amiodarone, budesonide, buprenorphine, buspirone, Calcium Channel Blockers, carbamazepine, cisapride, clarithromycin, clonazepam, cocaine, cortisol, cyclosporine, dexamethasone, erythromycin, fentanyl, ketoconazole, losartan, miconazole, midazolam, quinidine, sertraline, statins, tacrolimus, tamoxifen, TCAs, triamzolam, zolpidem, or mixtures thereof.

Additional examples of drug classes and drugs that can be employed in tablets of the present invention include:
antihistamines (e.g., azatadine maleate, brompheniramine maleate, carbinoxamine maleate, chlorpheniramine maleate, dexchlorpheniramine maleate, diphenhydramine hydrochloride, doxylamine succinate, methdilazine hydrochloride, promethazine, trimeprazine tartrate, tripelennamine citrate, tripelennamine hydrochloride and triprolidine hydrochloride);
antibiotics (e.g., penicillin V potassium, cloxacillin sodium, dicloxacillin sodium, nafcillin sodium, oxacillin sodium, carbenicillin indanyl sodium, oxytetracycline hydrochloride, tetracycline hydrochloride, clindamycin phosphate, clindamycin hydrochloride, clindamycin palmitate HCL, lincomycin HCL, novobiocin sodium, nitrofurantoin sodium, metronidazole hydrochloride); antituberculosis agents (e.g., isoniazid);
cholinergic agents (e.g., ambenonium chloride, bethanecol chloride, neostigmine bromide, pyridostigmine bromide);
antimuscarinics (e.g., anisotropine methylbromide, clidinium bromide, dicyclomine hydrochloride, glycopyrrolate, hexocyclium methylsulfate, homatropine methylbromide, hyoscyamine sulfate, methantheline bromide, hyoscine hydrobromide, oxyphenonium bromide, propantheline bromide, tridihexethyl chloride);
sympathomimetics (e.g., bitolterol mesylate, ephedrine, ephedrine hydrochloride, ephedrine sulphate, orciprenaline sulphate, phenylpropanolamine hydrochloride, pseudoephedrine hydrochloride, ritodrine hydrochloride, salbutamol sulphate, terbutaline sulphate);
sympatholytic agents (e.g., phenoxybenzamine hydrochloride); miscellaneous autonomic drugs (e.g., nicotine);
iron preparations (e.g., ferrous gluconate, ferrous sulphate);
haemostatics (e.g., aminocaproic acid);
cardiac drugs (e.g., acebutolol hydrochloride, disopyramide phosphate, flecainide acetate, procainamide hydrochloride, propranolol hydrochloride, quinidine gluconate, timolol maleate, tocainide hydrochloride, verapamil hydrochloride);
antihypertensive agents (e.g., captopril, clonidine hydrochloride, hydralazine hydrochloride, mecamylamine hydrochloride, metoprolol tartrate ); vasodilators (e.g., papaverine hydrochloride);
non-steroidal anti-inflammatory agents (e.g., choline salicylate, ibuprofen, ketoprofen, magnesium salicylate, meclofenamate sodium, naproxen sodium, tolmetin sodium);
opiate agonists (e.g., codeine hydrochloride, codeine phosphate, codeine sulphate, dextromoramide tartrate, hydrocodone bitartrate, hydromorphone hydrochloride, pethidine hydrochloride, methadone hydrochloride, morphine sulphate, morphine acetate, morphine lactate, morphine meconate, morphine nitrate, morphine monobasic phosphate, morphine tartrate, morphine valerate, morphine hydrobromide, morphine hydrochloride, propoxyphene hydrochloride);
anticonvulsants (e.g., phenobarbital sodium, phenytoin sodium, troxidone, ethosuximide, valproate sodium);
tranquilizers (e.g., acetophenazine maleate, chlorpromazine hydrochloride, fluphenazine hydrochloride, prochlorperazine edisylate, promethazine hydrochloride, thioridazine hydrochloride, trifluoroperazine hydrochloride, lithium citrate, molindone hydrochloride, thiothixine hydrochloride);
chemotherapeutic agents (e.g., doxorubicin, cisplatin, floxuridine, methotrexate, combinations thereof);
lipid lowering agents (e.g., gemfibrozil, clofibrate, HMG-CoA reductase inhibitors, such as for example, atorvastatin, cerivastatin, fluvastatin, lovastatin, pravastatin, simvastatin);
H.sub.2-antagonists (e.g., cimetidine, famotidine, nizatidine, ranitidine HCl);
anti-coagulant and anti-platelet agents (e.g., warfarin, cipyridamole, ticlopidine);
bronchodilators (e.g., albuterol, isoproterenol, metaproterenol, terbutaline);
stimulants (e.g., benzamphetamine hydrochloride, dextroamphetamine sulphate, dextroamphetamine phosphate, diethylpropion hydrochloride, fenfluramine hydrochloride, methamphetamine hydrochloride, methylphenidate hydrochloride, phendimetrazine tartrate, phenmetrazine hydrochloride, caffeine citrate);
barbiturates (e.g., amylobarbital sodium, butabarbital sodium, secobarbital sodium);
sedatives (e.g., hydroxyzine hydrochloride, methprylon); expectorants (e.g., potassium iodide);
antiemetics (e.g., benzaquinamide hydrochloride, metoclopropamide hydrochloride, trimethobenzamide hydrochloride);
gastro-intestinal drugs (e.g., ranitidine hydrochloride); heavy metal antagonists (e.g., penicillamine, penicillamine hydrochloride);
antithyroid agents (e.g., methimazole);
genitourinary smooth muscle relaxants (e.g., flavoxate hydrochloride, oxybutynin hydrochloride);
vitamins (e.g., thiamine hydrochloride, ascorbic acid);
unclassified agents (e.g., amantadine hydrochloride, colchicine, etidronate disodium, leucovorin calcium, methylene blue, potassium chloride, pralidoxime chloride.
steroids, particularly glucocorticoids (e.g., prednisolone, methylprednisolone, prednisone, cortisone, hydrocortisone, methylprednisolone, betamethasone, dexamethasone, triamcinolone).

Notwithstanding the general applicability of the tablets in relation to a wide range of drug substances, the present invention is particularly suited to delivery of the glucocorticosteroids aforementioned, and particularly prednisone, prednisolone and methylprednisolone. These steroids are useful in the treatment of *i.a*. of rheumatoid arthritis and joint pain.

As used above, prednisone refers to the compound and its salts or derivatives thereof, including prednisone 21 acetate.

As used above, prednisolone refers to the compound and its salts or derivatives including the 21-acetate, its 21-tert-butyl acetate, 21-succinate sodium salt, 21-stearoylglycolate, 21-m-sulphobenzoate sodium salt, and its trimethylacetate.

Methylprednisolone, as used above refers to the compound or and its salts and derivatives thereof including its 21 acetate, 21-phosphate disodium salt, 21-succinate sodium salt, and its acetonate.

Typically a core may contain about 0.1 to 50% by weight, more particularly 1 to 20%, still more particularly 1 to 10% by weight of active substance based on the total weight of the core.

The amount of glucocorticosteroid employed in tablets of the present invention will depend on the particular compound used, and the nature and severity of the condition to be treated. Typically a core may contain about 1 to 10% by weight of steroid. In the case of prednisone, it may be employed in amounts to provide a total weight per unit dosage form of 1 or 5mg, to offer convenience and flexibility of dosing, although dosage forms containing larger or smaller amounts of drug substance could be employed if desired.

These glucocorticosteroids are useful in the treatment of a variety of conditions such as rheumatoid arthritis, allergies, asthma or intestinal conditions such as Crohn's disease or ulcerative colitis.

Similarly, any conventional tabletting excipients may be employed in both core and coating. The particular core and coating excipients employed will depend on the particular release rate of active agent substance that is desired to be achieved.

Core materials may be chosen to provide an immediate release effect upon contact with moisture and as such may contain any of the known disintegrating or effervescing excipients to achieve this purpose. Alternatively, the skilled person may wish to have a slow release of active agent and therefore employ excipients, or mixtures of excipients that form a gel matrix when contacted with physiological media, thereby to permit of slow diffusion of the active substance in a sustained release manner.

In one embodiment useful in providing a rapid release of active agent from the core, the core may contain a disintegrating agent or mixtures of disintegrating agents. The disintegrating agents useful in the exercise of the present invention may be materials that effervesce in the presence of aqueous media thereby to provide the force necessary to mechanically disrupt the coating material. The disintegrants do not or substantially do not swell or gel and prevent release of the active agent. Preferably a core contains, in addition to the active agent, cross-linked polyvinyl pyrollidone and croscarmellose sodium. Cross-linked polyvinyl pyrollidone is described above, and may be employed in the core in the amounts disclosed in relation to the core. Croscarmellose sodium is an internally cross-linked sodium carboxymethyl cellulose (also known as Ac-Di-Sol). It may be used in amounts of 5 to 30% by weight based on the core, preferably 10 to 25%, e.g. 15 to 20% by weight.

The core may additionally comprise common tablet excipients such as those described in relation to the coating material.

Coating materials may be chosen that simply provide a water-insoluble or poorly soluble coating that delays ingress of physiological media into the core; and the coating may contain hydrophilic or hydrophobic excipients to either increase or decrease the rate of ingress. Other coating materials may be employed that degrade upon contact with physiological environments of certain pH values, or in response to the action of physiological reactive media such as enzymes. Other coating materials may be employed that simply provided an aesthetic aspect such as pleasant tasting material excipients. Coatings may even contain colourants for aesthetic purposes or to provide a visual cue to a patient in selecting the appropriate medicament. However, the nature and amount of colourant should be selected such that there is sufficient contrast between the coating colourant and core colourant or excipients to ensure the core can, be detected by the visible radiation, or higher energy radiation means such as x-rays.

In one embodiment, the coating is water insoluble or substantially water insoluble, and is hydrophobic. In this embodiment, the coating may contain one or more of the excipients selected from cellulose derivatives such as hydroxypropyl cellulose, hydroxypropylmethyl cellulose, carboxymethyl cellulose, and derivatives thereof; fatty acids or their esters or salts; long chain fatty alcohols; polyoxyethylene alkyl ethers; polyoxyethylene stearates; sugar esters; lauroyl macrogol-32 glyceryl, stearoyl macrogol-32 glyceryl, and the like. The coating contains one or more hydrophobic agents. As hydrophobic agents there may be mentioned any waxy substance known for use as a tablet excipient and having an HLB value of less than 5, and preferably about 2. Suitable hydrophobic agents include waxy substances such as carnauba wax, paraffin, microcrystalline wax, beeswax, cetyl ester wax and the like; or non-fatty hydrophobic substances such as calcium phosphate salts, e.g. dibasic calcium phosphate. Preferably the coating contains a calcium phosphate salt, glyceryl behenate, and polyvinyl pyrollidone, or mixtures thereof.

The polyvinyl pyrollidone is preferably present in amounts of about 1 to 25% by weight of the coating, more particularly 4 to 12%, e.g. 6 to 8%.

Glyceryl behenate, may be present as its mono-, di-, or tri-ester, and preferably the tri-ester or a mixture thereof, and is preferably present in amounts of about 5 to 85% by weight of the coating, more particularly from 10 to 70% by weight, e.g. 5 to 10%.

The calcium phosphate salt may be the dibasic calcium phosphate dihydrate, and may be present in an amount of about 10 to 90% by weight of the coating, preferably 20 to 80%, e.g. 40 to 75%.

The coating may contain other common tablet excipients such as lubricants, colourants, binders, diluents, glidants and taste-masking agents or flavourants.
Examples of excipients include colourants such a ferric oxide, e.g. yellow ferric oxide; lubricants such as magnesium stearate; and glidants such as silicon dioxide, e.g. colloidal silicon dioxide. Yellow ferric oxide may be used in amounts of about 0.01 to 0.5% by weight based on the coating; magnesium stearate may be present in amounts of 1 to 20% by weight of the coating; and colloidal silica may be used in amounts of 0.1 to 20% by weight of the coating.

The invention provides in another aspect, a method of forming tablets as herein above described. The tablets may be formed on conventional press coating equipment. A series of die are arrayed on a rotating platform. The die are removably mounted in the platform such that differently sized die may be employed as appropriate. Each die is hollow to receive a lower punch. The punch is positioned within the die such that the upper surface of the punch and the inner surface of the die define a volume for receiving a precise amount coating material. Once loaded, die is rotated on the platform until it is positioned under an upper punch. The upper punch is then urged down onto the coating material and the coating material is pre-compressed or tamped between the upper and lower punch. A pre-formed core is then fed into die to rests on the tamped coating. Conventional press coating apparatus are equipped with centering devices that enable cores to be positioned both vertically and radially. This might be achieved by a tamping process, whereby an initial amount of coating material is placed in a die and is tamped with a shaped punch that leaves an indentation in the coating material in which to receive a core. Thereafter, in a second filling operation, a precise amount of coating material is fed into the die to cover the core, and an upper punch compresses the coating material to form tablets according to the present invention.

The compression force applied during the tamping process is light and is just sufficient to provide a bed for the core and to prevent movement of the coating material as a result of centrifugal force. Subsequent compression to form the tablet may be adjusted to give tablets of requisite hardness. Preferably, this compression force is 400 kg, although this may be adjusted by +/- 30% in order to give tablets of the required hardness.

The amount of coating material fed into the die can be precisely defined having regard to the density of the coating material to ensure, after compression that the tablet is formed with the required coating thickness. Should it be necessary to change the thickness of the coating, die of appropriate internal dimensions may be placed in the rotating platform, and the amount of coating material fed into the die may be changed accordingly.

Suitable rotary tablet machines having high process speeds are known in the art and need no further discussion here.

The hardness of the tablet is preferably at least 60 Newtons, e.g. 60 to 80 Newtons, and more particularly 60 to 75 Newtons. Hardness may be measured according to a process described in The European Pharmacopoeia 4, 2.9.8 at page 201. The test employs apparatus consisting of 2 opposing jaws, one of which moves towards the other. The flat surfaces of the jaws are perpendicular to the direction of movement. The crushing surfaces of the jaws are flat and larger than the zone of contact with the tablet. The apparatus is calibrated using a system with a precision of one Newton. The tablet is placed between the jaws. For each measurement, the tablet is oriented in the same way with respect to the direction of the applied force. Measurements are carried out on 10 tablets. Results are expressed in terms of the mean, minimum and maximum values (in Newtons) of the force needed to crush the tablets.

Tablets having a hardness within this range are mechanically robust to withstand forces generated in the stomach, particularly in the presence of food. Furthermore, the tablets are sufficiently porous to permit ingress of aqueous media to the core.

The cores may likewise be formed using a conventional rotary tablet machine. Cores are preferably compressed under compression forces sufficient to provide cores having a hardness of about 60 Newtons or more, e.g. 50 to 70 Newtons. Cores having hardness in this range give desired release characteristics. If desired, the cores can be formed at the same time as the tablets are produced. In such case, one might employ a Manesty Dry Cota. Such a press consists of two side-by-side and inter-connected presses where the core is made on one press before being mechanically transferred to the other press for compression coating. Such equipment and techniques for making tablets using such equipment is known in the art and no more needs to be said about this here.

Cores are preferably formed according to wet granulation techniques generally known in the art. In a typical procedure, core materials are sieved and blended. Granulating fluid, typically water is then added to the blend and the mixture is homogenized to form a granulate, which is then sprayed dried or dried on a fluid bed drier to obtain a granulate with requisite residual moisture. Preferably the residual moisture content is from about 0.4 to 2.0% by weight. The granulate is then sized by passing it through screens of desired aperture. At this stage, any adjuvants are sized and added to the granulate to form the core composition suitable for compression. The skilled person wil appreciate that a coating composition can be formed in an analogous manner. The skilled person will appreciate that granulates may be obtained having a range of particle sizes. It is preferred that the coating granulate has a fine fraction that is less than 30%. By "fine fraction" is meant granulate having particle size of up to about 63 microns.

Preferred features of the second and subsequent aspects of the invention may be as for the first aspect *mutatis mutandis*.

There now follows an example that serves to illustrate the invention.

### Example 1

### (Preparation of a tablet)

### Core:

Prednisone 8.33%
Lactose monohydrate 64.47%
Povidone 6.67%
Croscarmellose sodium 18.33%
Red ferric oxide 0.5%
Magnesium stearate 1.0%
Colloidal silicon dioxide 0.5%

### Coating

Dibasic calcium phosphate dihydrate 50%
Glyceryl behenate 40%
Povidone 8.40%
Yellow ferric oxide 0.1 %
Magnesium stearate 1.0%
Colloidal silicon dioxide 0.5%

The core was prepared for the press coated system as follows.

The required amounts of prednisone, and other ingredients (ex silicon dioxide and magnesium stearate) were weighed and manually sieved with a screen having 0.710 mm apertures. The components were homogeneously mixed in a Niro-Fielder PMA 25-litre mixing granulator for 6 min at impeller speed 250 rpm without chopper. A prednisone assay was performed on this premix. Subsequently, the granulating solution (purified water, 25.47 % of the weight of the dry blend was added within 4 min at impeller speed 250 rpm and chopper speed 1500 rpm, using a nozzle H1/4VV-95015 (spraying rate of 250 g/min). Mixing was continued for homogenisation and massing of the wet mass for 3 min at impeller speed 500 rpm and chopper speed 3000 rpm.

The mixed wet granulate was then dried in a Glatt WSG5 fluidised air bed drier. The inlet temperature was maintained at 45°C during drying. The drying lasted 20 min to get a granulate with a residual moisture less than 2.5%. The yielded dry granulate was calibrated in a Frewitt MGI 205 granulator using a screen with 0.8 mm apertures for 3 min at speed 244 osc/min (graduation 7). Appropriate amounts of Aerosil^{®} 200 and magnesium stearate were manually sieved using a screen with 1.0 mm apertures. Half of the dry granulate was put in a Niro-Fielder PMA 25-litre mixing granulator, followed by Aerosil^{®} 200 and then by the other half of the dry granulate. The ingredients were mixed for 2 min at impeller speed 250 rpm. Finally, magnesium stearate was added and mixing was continued for 2 min at impeller speed 250 rpm.

The coating was prepared according to the process described below.

Weighed amounts of ingredients (ex magnesium stearate and silicon dioxide) were manually sieved with a screen having 0.710 mm apertures. They were placed in a Niro-Fielder PMA 65-litre mixing granulator. Then, the components were homogeneously mixed for 6 min, at impeller speed 200 rpm, without chopper. Subsequently, the granulating solution (purified water, 8.12 % of the weight of the dry blend) was added within 2 min at impeller speed 200 rpm and chopper speed 1500 rpm using a nozzle 4,9 (spraying rate of 520 g/min). Mixing was continued for homogenisation and massing for 1 min at impeller speed 400 rpm and chopper speed 3000 rpm.

The mixed wet granulate was then dried in a Niro-Fielder TSG 2 fluidised air bed dryer. The inlet temperature was maintained at 45°C during drying. The drying lasted 33 min to have residual moisture less than 2.5%. The yielded dry granulate was calibrated in a Frewitt MGI 205 granulator using a screen having 0.8 mm apertures for 4 min at speed 244 osc/min (graduation 7). Appropriate amounts of silicon dioxide (Aerosil^{®} 200) and magnesium stearate were manually sieved using a screen with 1.0 mm apertures. Half of the dry granulate was put in a Niro-Fielder PMA 65-litre, followed by Aerosil^{®} 200 and then by the other half of the dry granulate. The ingredients were mixed for 2 min at impeller speed 200 rpm, without chopper. Finally, magnesium stearate was added and mixing was continued for 2 more minutes at impeller speed 200 rpm, without chopper.

The coating was press coated on active-containing core to have press coated tablets. Press coating was carried out utilising a Kilian RUD tabletting machine. First and second loading hoppers are filled up with the coating granulate. Between the two loading hoppers, the machine is equipped with a transfer system adapted to feed the cores. For each tablet, the first loading hopper supplies with about half of the quantity to be applied to the core. Then, the feeding system provides and positions a core centred in the die. Subsequently, the second loading hopper supplies with the other half of the quantity to be applied to the core. The compression step then occurs.

### Equipment implemented for the manufacturing process

| Equipment | Brand name/Type | Manufacturer/Supplier |
|---|---|---|
| Mixing granulator | Niro-Fielder PMA 25/65 litres | Aéromatic-Fielder AG, Bubendorf, Switzerland |
| Fluidised air bed dryer | Glatt WSG5 | Maschinen und apparatebau AG, Pratteln, Switzerland |
| Fluidised air bed dryer | Niro-Fielder TSG 2 | Aéromatic-Pielder AG, Bubendorf, Switzerland |
| Granulator | Frewitt MGI 205 | Frewitt SA, Granges-Pacot, Switzerland |
| Infrared moisture analyser | Mettler PE 360 Moisture Analyzer | Mettler Toledo AG, Greifensee, Switzerland |
| Multilayer tablet press | Hata HT-AP55LS-U/3L | Elisabeth-Carbide, Antwerp, Belgium |
| Dry coating tablet press | Kilian RUD | Kilian & Co GmbH, Cologne, Germany |
| | | |

## Claims

1. A tablet comprising a core containing an active substance and a compression coating surrounding the core, wherein the core contains a colourant selected from the group consisting of titanium dioxide, calcium sulphate, magnesium oxide, aluminium silicate, aluminium hydroxide and iron oxide or an excipient that is opaque to x-rays such that when the tablet is exposed to penetrating radiation the core is contrasted with the coating and is visible through the coating.

2. A tablet according to claim 1 wherein the core contains a colourant that is red iron oxide.

3. A tablet according to claim 1 wherein the excipient is barium sulphate.

4. A tablet according to any of the preceding claims wherein the colourant is present in amounts of 0.1 % to 1.75% by weight based on the total amount of the tablet.

5. A tablet according to claim 3 wherein the excipient is present in amounts of 0.1 to 2% based on the weight of the tablet.

6. A tablet according to any of the preceding claims wherein the active substance is a glucocorticosteroid selected from prednisone, prednisolone or methylprednisolone.

7. A tablet according to any of the preceding claims in unit dosage form containing 1 or 5 mg prednisone.

8. A method of forming a tablet as defined in any of the preceding claims comprising the step of compressing coating material in granulate powder form around the core.

9. The use of a colouring agent selected from the group consisting of titanium dioxide, calcium carbonate, calcium sulphate, magnesium oxide, aluminium silicate, aluminium hydroxide, talc and iron oxide or an excipient opaque to x-ray radiation in the core of a press-coated tablet comprising a core containing an active substance, and a coating surrounding said core, as a means for determining that the core is correctly located within the coating.

## Patentansprüche

1. Tablette, umfassend einen Kern, der eine Wirksubstanz enthält, und eine den Kern umgebende Pressbeschichtung, wobei der Kern einen Farbstoff, der aus der Gruppe bestehend aus Titandioxid, Calciumsulfat, Magnesiumoxid, Aluminiumsilicat, Aluminiumhydroxid und Eisenoxid ausgewählt ist, oder einen Exzipienten enthält, der gegenüber Röntgenstrahlen undurchlässig ist, so dass der Kern, wenn die Tablette einer durchdringenden Strahlung ausgesetzt ist, mit der Beschichtung kontrastiert wird und durch die Beschichtung hindurch sichtbar ist.

2. Tablette nach Anspruch 1, wobei der Kern einen Farbstoff enthält, der rotes Eisenoxdid ist.

3. Tablette nach Anspruch 1, wobei der Expzipient Bariumsulfat ist.

4. Tablette nach einem der vorhergehenden Ansprüche, wobei der Farbstoff basierend auf der Gesamtmenge der Tablette in Mengen von 0,1 Gew.-% bis 1,75 Gew.-% vorhanden ist.

5. Tablette nach Anspruch 3, wobei der Exzipient basierend auf dem Gewicht der Tablette in Mengen von 0,1 % bis 2 % vorhanden ist.

6. Tablette nach einem der vorhergehenden Ansprüche, wobei die Wirksubstanz ein Glucocorticosteroid ist, ausgewählt aus Prednison, Prednisolon oder Methylprednisolon.

7. Tablette nach einem der vorhergehenden Ansprüche in einer Einheitsdosierungsform, die 1 bis 5 mg Prednison enthält.

8. Verfahren zum Bilden einer Tablette nach einem der vorhergehenden Ansprüche, umfassend den Schritt des Pressens von Beschichtungsmaterial in granulierter Pulverform um den Kern.

9. Verwendung eines Farbstoffes, der aus der Gruppe bestehend aus Titandioxid, Calciumcarbonat, Calciumsulfat, Magnesiumoxid, Aluminiumsilicat, Aluminiumhydroxid, Talk und Eisenoxid ausgewählt ist, oder eines Exzipienten, der gegenüber Röntgenstrahlen undurchlässig ist, im Kern einer pressbeschichteten Tablette, die einen Kern, der eine Wirksubstanz enthält, und eine den Kern umgebende Beschichtung umfasst, als Mittel zum Bestimmen, ob der Kern in der Beschichtung korrekt angeordnet ist.

## Revendications

1. Comprimé comprenant un noyau contenant une substance active et un enrobage par compression entourant le noyau, dans lequel le noyau contient un colorant choisi dans le groupe comprenant le dioxyde de titane, le sulfate de calcium, l'oxyde de magnésium, le silicate d'aluminium, l'hydroxyde d'aluminium et l'oxyde de fer ou un excipient qui est opaque aux rayons X de sorte que lorsque le comprimé est exposé à la pénétration des rayons, le noyau contraste avec l'enrobage et soit visible à travers l'enrobage.

2. Comprimé selon la revendication 1, dans lequel le noyau contient un colorant qui est l'oxyde de fer rouge.

3. Comprimé selon la revendication 1, dans lequel l'excipient est le sulfate de baryum.

4. Comprimé selon l'une quelconque des revendications précédentes, dans lequel le colorant est présent en quantités de 0,1 % à 1,75 % en poids sur la base de la quantité totale du comprimé.

5. Comprimé selon la revendication 3, dans lequel l'excipient est présent en quantités de 0,1 à 2 % sur la base en poids du comprimé.

6. Comprimé selon l'une quelconque des revendications précédents, dans lequel la substance active est un glucocorticostéroïde choisi parmi la prednisone, la prednisolone ou la méthylprednisolone.

7. Comprimé selon l'une quelconque des revendications précédentes, sous forme galénique unitaire contenant 1 à 5 mg de prednisone.

8. Procédé de formation d'un comprimé tel que défini dans l'une quelconque des revendications précédentes, comprenant l'étape de compression du matériau d'enrobage sous forme de poudre granulée autour du noyau.

9. Utilisation d'un agent colorant choisi dans le groupe comprenant le dioxyde de titane, le carbonate de calcium, le sulfate de calcium, l'oxyde de magnésium, le silicate d'aluminium, l'hydroxyde d'aluminium, le talc et l'oxyde de fer ou un excipient opaque aux rayons X dans le noyau d'un comprimé enrobé par compression, comprenant un noyau contenant une substance active et un enrobage entourant ledit noyau, en tant que moyen pour déterminer que le noyau est correctement positionné dans l'enrobage.
